# EUROPEAN PATENT SPECIFICATION

(11) **EP 1 207 857 B1**
(45) Date of publication and mention of the grant of the patent: **04.06.2003**
(21) Application number: 00954808.2
(22) Date of filing: 11.08.2000
(51) Int. Cl.: A61K 9/20, A61K 9/36, A61K 31/497

(54) **CIPROFLOXACIN CONTAINING PHARMACEUTICAL COMPOSITION AND PROCESS FOR THE PREPARATION THEREOF**
CIPROFLOXACIN ENTHALTENDE PHARMAZEUTISCHE ZUSAMMENSETZUNG UND VERFAHREN ZU DEREN HERSTELLUNG
COMPOSITION PHARMACEUTIQUE A BASE DE CIPROFLOXACINE ET SON PROCEDE DE PREPARATION

(30) Priority: 11.08.1999 HU 9902725
(43) Date of publication of application: 29.05.2002
(73) Proprietor: EGIS GYOGYSZERGYAR RT., 1106 Budapest (HU)
(72) Inventor: FEKETE, Pál, H-1141 Budapest (HU); FELLNER, Györgyné, H-1122 Budapest (HU); GORA, Lászloné, H-2117 Isaszeg (HU); JAMBOR, Istvánné, H-1182 Budapest (HU); FEIKUS, Szilvia, H-1042 Budapest (HU); PALFI, Zoltánné, H-1172 Budapest (HU); ZSIGMOND, Zsolt, H-1173 Budapest (HU)
(74) Representative: Cabinet Hirsch
(86) International application number: HU0000090
(87) International publication number: WO01012162

(56) References cited:
- EP-A- 0 230 881
- WO-A-98/01114
- WO-A-98/06385

## Description

### Technical background

This invention relates to ciprofloxacin containing pharmaceutical compositions and a process for the preparation thereof. More particularly the present invention is concerned with immediate release ciprofloxacin containing pharmaceutical compositions and a process for the preparation thereof.

### State of the art

Ciprofloxacin is a broad spectrum bactericidal chemotherapeutical agent belonging to the family of fluoro quinolines (gyrase inhibitors). The chemical formula of ciprofloxacin is 1-cyclopropyl-6-fluoro-1,4-dihydro-4-oxo-7-(1--piperazinyl)-quinoline-3-carboxylic acid and is used in the form of ciprofloxacin monohydrochloride monohydrate (HU 187,580).

It is known that after oral administration ciprofloxacin is quickly absorbed from the gastrointestinal system. During absorption the active ingredient is not metabolized in the liver to a considerable extent and for this reason bioavailability is about 70 %. The serum concentration increases proportionally to the dose and the maximal serum concentration is achieved 1-2 hours after administration. In individuals with healthy kidney the half time of the serum concentration amounts to about 4 hours.

In view of the above pharmacokinetical properties of ciprofloxacin immediate release of the active ingredient is a very important requirement so that the dosage form should not delay the absorption of the active ingredient.

Empirical experiences showed that the preparation of ciprofloxacin containing pharmaceutical compositions with immediate release of the active ingredient is not an easy task. On adapting the composition of tablets containing norfloxacin [1-ethyl-6-fluoro-1,4-dihydro-4-oxo-7-(1-piperazinyl)-quinoline--3-carboxylic acid, i.e. an active ingredient chemically closely related to ciprofloxacin; see GB 2,160,519] to ciprofloxacin, tablets with a very slow active ingredient release are obtained (HU 196,710).

The pharmaceutical compositions described in GB 2,160,519 contain polyvinyl pyrrolidone or microcrystalline cellulose as binder, the surfactant sodium lauryl sulfate as dissolution accelerator and magnesium stearate as lubricant. The preparation of ciprofloxacin containing pharmaceutical compositions with immediate release is disclosed in HU 196,710. The pharmaceutical compositions described in this patent specification contain in addition to the ciprofloxacin active ingredient a dry (water insoluble) binder based on microcrystalline cellulose; a disintegrating agent based on starch, a cellulose derivative and/or polyvinyl pyrrolidone and a lubricant. Although the dissolution of the ciprofloxacin tablets disclosed in HU 196,710 is quicker than that of the tablets prepared according to GB 2,160,159, the release rate is just higher than 50 % after 5 minutes.

The release rate of the ciprofloxacin containing pharmaceutical compositions prepared according to GB 2,160,519 and HU 196,710 is shown in Table I.

**Table I**

| ***Prior art*** | ***5 minutes*** | ***10 minutes*** | ***15 minutes*** | ***30 minutes*** |
|---|---|---|---|---|
| GB 2 160 519 (Example 1) | 3% | 7% | 9% | 13% |
| HU 196 710 (Example 1) | 51% | 81% | 92% | 96% |

WO-A-9801114 discloses fast-disintegrating and fast-dissolving tablets comprising cyprofloxacin, dispersible cellulose (microcrystalline cellulose and carboxymethyl cellulose), microcrystalline cellulose, cross-linked polyvinylpyrrolidone and a lubricant.

### Description of the invention

It is the object of the present invention to provide alternative ciprofloxacin containing pharmaceutical compositions which have a suitable hardness and show a quick active ingredient release.

The above object is achieved by means of the present invention.

The present invention relates to an immediate release pharmaceutical composition containing ciprofloxacin, a binder, disintegrating agent and optionally further pharmaceutical auxiliary agents which comprises - related to the total weight of the composition - 60-80 % by weight of ciprofloxacin in the form of ciprofloxacin hydrochloride monohydrate, 2-10 % by weight of maltodextrin, 5-15 % by weight of a disintegrating agent of carboxy methyl starch type, 3-6 % by weight of silicium dioxide, 1-3 % by weight of a lubricant and - in case of film-coated tablets - 2-6 % of a film coating layer.

The present invention is partly based on the surprising recognition that disintegrating agents based on carboxymethyl starch have a very favourable effect on the dissolution of ciprofloxacin active ingredient.

The effect of disintegrating agents of different types on the dissolution of ciprofloxacin tablets is shown in Table II. The composition of the tablets and also the breaking strength and disintegration time of the tablets measured by using a compression force of 20 kN is disclosed in the Table. The tablets are prepared by admixing ciprofloxacin active ingredient suitable for direct pressing with the further auxiliary agents in a gravitation type mixer. The powder mixture is pressed into tablets weighing 367 mg on a Fette E Xl single punch machine by using a concave tool having a diameter of 11 mm. Each tablet contains 275 mg of ciprofloxacin.

**Table II**

| Effect of the disintegrating agents on the dissolution of ciprofloxacin (direct pressing - without binder) | | | | | | | | |
|---|---|---|---|---|---|---|---|---|
| | Disintegrating agent according to HU 196,710 | | | | | Other disintegrating agents | | |
| No. of experiment | 1 | 2 | 3 | 4 | 5 | 6 | 7 | 8 |
| Ciprofloxacin hydrochloride monohydrate | 291.0 | 291.0 | 291.0 | 291.0 | 291.0 | 291.0 | 291.0 | 291.0 |
| Magnesium stearate | 3.0 | 3.0 | 3.0 | 3.0 | 3.0 | 3.0 | 3.0 | 3.0 |
| 1/ Crospovidon | 40.0 | | | | | | | |
| 2/ Sodium carboxymethyl cellulose | | 40.0 | | | | | | |
| 3/ Maize starch | | | 40.0 | | | | | |
| 4/ Starch 1500 | | | | 40.0 | | | | |
| 5/ L-HPC | | | | | 40.0 | | | |
| 6/ Guar gum | | | | | | 40.0 | | |
| 7/ Sodium alginate | | | | | | | 40.0 | |
| 8/ Sodium carboxymethyl starch | | | | | | | | 40.0 |
| Breaking strength (N) | **81.7** | **70.7** | 27.9 | 18.7 | **79.4** | 20.1 | 33.4 | 14.6 |
| Disintegration time (minutes) | 10.4 | 1.0 | 13.9 | 6.7 | 3.1 | 4.9 | 45.0 | **0.4** |
| Crospovidon = cross-linked polyvinyl pyrrolidone, HPE: page 143, | | | | | | | | |
| Starch 1500 = partially hydrolysed maize starch, HPE: page 491, | | | | | | | | |
| L-HPC = hydroxy propyl cellulose with low substitution degree, HPE: page 427. | | | | | | | | |

The above auxiliary agents are described in details on the indicated pages of "Handbook of Pharmaceutical Excipients" (HPE) 2^{nd} edition (1994), The Pharm. Press, London (editor: A. Wade and P. Weller).

It can be clearly seen from the results of Table II that in the absence of a binder tablets of suitable hardness can only be prepared by using the disintegrating agents suggested in HU 196,710, namely Crospovidon, based on polyvinyl pyrrolidone (Experiment No. 1), sodium carboxy methyl cellulose, based on a cellulose derivative (Experiment No. 2) and L-HPC, based on hydroxy propyl cellulose of a low substitution degree (Experiment No. 5).

The quickest disintegration is obtained by using a disintegrating agent based on carboxy methyl starch (Experiment No. 8). However, the breaking strength of the tablets thus obtained is very low. Tablets prepared by using the other disintegrating agents (maize starch, partially hydrolysed starch, sodium alginate and guar gum) do not meet the requirements either from the point of view of the disintegrating time or from that of the hardness of the tablets.

The present invention is also based on the further surprising recognition that disintegrating agents based on carboxy methyl starch exhibit a highly favourable effect on the dissolution of ciprofloxacin not only in the absence of binders but also in the presence of water soluble binders which provide a suitable hardness to the tablets. This recognition is so much the more surprising as disintegrating agents disclosed in HU 196,710 [cellulose derivatives, such as sodium carboxy methyl cellulose, low substitution degree hydroxy propyl cellulose (L-HPC), polyvinyl pyrrolidone derivatives (e.g. Crospovidon) and other disintegrating agents, e.g. sodium alginate or guar gum] failed to be sufficiently effective in the presence of water soluble (wet) binders.

On the contrary, it has been found that on using simultaneously maltodextrin as binder and a disintegrating agent based on carboxy methyl starch, ciprofloxacin containing tablets are obtained which have excellent breaking strength and show an extremely quick active ingredient release.

The results are summarized in Table III.

By the preparation of the tablets maltodextrin is used as binder. The components disclosed in Table III are admixed dry - with the exception of magnesium stearate -, the mixture is kneaded under wetting with water, dried, sieved, homogenized with magnesium stearate and pressed in tablets weighing 374 mg on a Fette E XI single punch machine by using a concave tool which has a diameter of 11 mm. Each tablet contains 250 mg of ciprofloxacin.

**Table III**

| Effect of disintegrating agents on the dissolution of ciprofloxacin (in the presence of wet granulating binders) | | | | | |
|---|---|---|---|---|---|
| No. of experiment | 9 | 10 | 11 | 12 | 14 |
| Ciprofloxacin hydrochloride monohydrate | 291.0 | 291.0 | 291.0 | 291.0 | 291.0 |
| Maltodextrin | 30.0 | 30.0 | 30.0 | 30.0 | 30.0 |
| Silicium dioxide | 12.0 | 12.0 | 12.0 | 12.0 | 12.0 |
| Magnesium stearate | 3.0 | 3.0 | 3.0 | 3.0 | 3.0 |
| 9/ Crospovidon | 38.0 | | | | |
| 10/ L-HPC | | 38.0 | | | |
| 11/ Sodium alginate | | | 38.0 | | |
| 12/ Guar gum | | | | 38.0 | |
| 13/ Sodium carboxy methyl starch | | | | | 38.0 |
| Breaking strength (N) | 111.1 | 71.2 | 103.7 | 112.6 | 110.6 |
| Disintegration time (minutes) | 26.0 | 9.7 | 38.8 | 15.7 | 7.1 |

| Dissolution (%) | | | | | |
|---|---|---|---|---|---|
| 5 minutes | 15.0 | 31.7 | 16.4 | 18.6 | **87.6** |
| 10 minutes | 22.9 | 52.0 | 23.8 | 43.4 | **96.0** |
| 15 minutes | 31.7 | 76.0 | 36.6 | 61.6 | **97.0** |
| 30 minutes | 49.7 | 96.0 | 66.0 | 78.3 | **99.0** |

The data of Table III clearly show that in the presence of maltodextrin as binder immediate release of the active ingredient can only be obtained by using sodium carboxy methyl starch as disintegrating agent. At the same time the breaking strength of such tablets prepared by using a disintegrating agent based on carboxy methyl starch is not lower than that of tablets containing disintegrating agents of different types. Moreover in some cases the breaking strength of the tablets of the present invention is even higher.

Accordingly the present invention is based on the recognition that pharmaceutical compositions containing ciprofloxacin, maltodextrin and a disintegrating agent based on carboxy methyl starch have very good mechanical strength, hardness and show a quicker active ingredient release than tablets prepared by prior art methods.

The pharmaceutical compositions of the present invention contain ciprofloxacin preferably in the form of ciprofloxacin hydrochloride monohydrate. Taking into consideration the high individual dose of ciprofloxacin (500 mg, i.e. 591 mg related to ciprofloxacin hydrochloride monohydrate), the active ingredient content of the pharmaceutical composition is high, namely 60-80 % by weight, related to the total weight of the composition.

The maltodextrin used as binder in the pharmaceutical compositions of the present invention is an oligosaccharide which can be prepared by hydrolysis of starch and forms a low viscosity colloidal solution in cold water. Maltodextrin is described in details in "Handbook of Pharmaceutical Excipients" (HPE) 2^{nd} edition (1994), The Pharm. Press, London (ed: A. Wade and P. Weller), pages 289-291.

The term "composition with immediate active ingredient release" relates to compositions from which at least 80 % of ciprofloxacin is released within 5 minutes when measured according to the method disclosed in USP 23, The United States Pharmacopoeia, 1995, page 379.

The pharmaceutical compositions according to the present invention contains sodium carboxy methyl starch as disintegrating agent based on carboxy methyl starch. Such disintegrating agents based on sodium carboxy methyl starch can be prepared from various types of starch of vegetable origin by partial carboxymethylation of the hydroxy groups of the polysaccharide chain or cross-linking by various degree, respectively. There are several commercially available disintegrating agents based on sodium carboxy methyl starch which differ from each other in water solubility, the pH of the aqueous extract and the drying losses. Sodium carboxy methyl starch is described in details in "Handbook of Pharmaceutical Excipients" (HPE) 2^{nd} edition (1994), The Pharm. Press, London (ed.: A. Wade and P. Weller), pages 462-466.

The pharmaceutical composition according to the present invention contains 3-6 % by weight of silicium dioxide. This is a hydrophilic fine granular substance insoluble in water. Silicium dioxide can be used in the composition of the present invention as precipitated product and also in the so-called colloidal form prepared by the pyrogenic process. It has been found that a composition having a higher breaking strength and a quicker active ingredient dissolution can be obtained by using 3-6 % of silicium dioxide, related to the total weight of the composition. It can be presumed that this might be due to the fact that silicium dioxide prevents the formation of hard compact granules during granulation. Colloidal silicium dioxide is described in details in "Handbook of Pharmaceutical Excipients" (HPE) 2^{nd} edition (1994), The Pharm. Press, London (ed.: A. Wade and P. Weller), pages 424-427.

In order to reduce friction during pressing of the tablets, the invention compositions may contain lubricants. For this purpose any suitable lubricant can be used, e.g. magnesium stearate, calcium stearate, stearic acid, hydrogenated vegetable oils, paraffin etc. One may preferably use magnesium stearate.

The pharmaceutical compositions of the present invention can be in the form of tablets or film-coated tablets. The latter dosage form contains a film-coating layer applied onto the tablets. Such compositions may contain preferably a film-coating based on water soluble hydroxy propyl methyl cellulose.

According to a further aspect of the present invention there is provided a process for the preparation of immediate release pharmaceutical composition which comprises admixing - related to the total weight of the composition -60-80 % by weight of in the form of ciprofloxacin hydrochloride monohydrate, 2-10 % by weight of maltodextrin, 5-15 % by weight of a disintegrating agent of the carboxy methyl starch type and 3-6 % by weight of silicium dioxide, granulating the mixture under the addition of water, drying, homogenizing with 1-3 % by weight of a lubricant, and pressing the mixture into tablets and, if desired, applying onto the surface of the tablets a film-coating layer in an amount of 2-6 % by weight.

The process of the present invention can be carried out as follows: granules are prepared from the ciprofloxacin active ingredient, maltodextrin, a disintegrating agent of the carboxy methyl starch type and silicium dioxide in a manner known per se by the "wet mixing" procedure. Granulation can be performed by means of the so-called "kneading" procedure with the aid of various mixers or by the "fluid-bed atomization" process. During granulation water is added to the powder mixture of the above components in a suitable mixer, or in the course of the fluid-bed atomization procedure water is sprayed until granules of the desired particle size are formed, whereupon the granules are dried in a manner known per se, the dried granules are sieved and, if desired, a film-coating layer is applied onto the surface of the tablets. The film-coating layer may be preferably a layer of the water soluble hydroxy propyl methyl cellulose type.

The process of the present invention may be preferably carried out in a manner known per se. Reference is made e.g. to Pharmaceutical Dosage Forms: Tablets, 2^{nd} edition, Marcel Dekker (1990); Remington's Pharmaceutical Sciences, 18^{th} edition, Mack Publ. Co. (1990).

Further details of the present invention are to be found in the following Examples.

### Example 1

### Fluidization granulation process

873 g of ciprofloxacin hydrochloride monohydrate and 60 g of colloidal silicium dioxide are sieved through a sieve (18 mesh) and the mixture is placed into the container of a fluidization spraying granulating equipment, type Glatt GPCG 1. To the mixture 60 g of maltodextrin and 123 g of sodium carboxy methyl starch are added. The mixture is homogenized with a fluidization air stream, granulated by spraying 700 g of water onto the mixture and dried. The granulate is sieved again, whereupon 9 g of magnesium stearate are added and the mixture is pressed into tablets having an average weight of 375 mg on a Manesty B3B type rotating tabletting machine by using a concave tool having a diameter of 11 mm.

The breaking strength of the tablets is 120.7 N.

The dissolution of the tablets is shown in the following Table IV.

**Table IV**

| 5 minutes | 10 minutes | 15 minutes | 30 minutes |
|---|---|---|---|
| 83.0 % | 90.8 % | 95.7 % | 98.9 % |

### Example 2

### Kneading granulation process

873 g of ciprofloxacin hydrochloride monohydrate, 60 g of colloidal silicium dioxide, 60 g of maltodextrin and 122 g of sodium carboxy methyl starch are placed into the container of granulating apparatus type Lödige M5R. The mixture is homogenized for 3 minutes, whereupon 400 g of water are added, the mixture is homogenized by kneading for 10 minutes and dried in a tray desiccator at 50°C. The granules are sieved on a sieve (18 mesh), whereupon 9 g of magnesium stearate are added and the mixture is then pressed into tablets having an average weight of 375 mg on a rotary tabletting machine type Manesty B3B by using a concave tool having a diameter of 11 mm.

The breaking strength of the tablets is 97.1 N.

In a coating apparatus type Glatt GC 250 a 10 % by weight aqueous suspension of the coating agent with the commercial name Opadry is applied onto the tablets. Thus a film--coating layer of 10 mg is formed on the surface of the tablets.

The dissolution of the film-coated tablets is shown in the following Table V.

**Table V**

| 5 minutes | 10 minutes | 15 minutes | 30 minutes |
|---|---|---|---|
| 81.3% | 94.6% | 97.7% | 98.8% |

### Example 3

### Tablets containing 500 mg of ciprofloxacin

The granules are prepared in accordance with Example 1 but the granules are pressed into tablets weighing 750 mg and containing 500 mg of ciprofloxacin on a tabletting machine type Kilian RTS 21D by using a 17 mm oblong tool.

The breaking strength of the tablets is 150 N.

The dissolution of the tablets is shown in Table VI.

**Table VI**

| 5 minutes | 10 minutes | 15 minutes | 30 minutes |
|---|---|---|---|
| 82.1 % | 93.8 % | 95.6 % | 96.1 % |

### Example 4

### Film-coated tablets containing 100 mg of ciprofloxacin

The granules are prepared in accordance with Example 2 but the granules are pressed into tablets weighing 150 mg by using a concave tool having a diameter of 9 mm, whereupon 4 mg of a white Opadry (manufacturer: Colorcon) coating layer are applied onto the surface of the tablets from an aqueous suspension.

The dissolution of the film-coated tablets is shown in Table VII.

**Table VII**

| 5 minutes | 10 minutes | 15 minutes | 30 minutes |
|---|---|---|---|
| 86.0 % | 97.0 % | 97.6 % | 98.1 % |

### Example 5

### Effect of the amount of the auxiliary agents used according to the present invention on the dissolution of ciprofloxacin

In an Erweka type laboratory kneading machine a mixture of 291 g of ciprofloxacin hydrochloride, colloidal silicium dioxide, sodium carboxy methyl starch and maltodextrin used in the amount disclosed in Table VIII is kneaded after addition of 160 g of water for 15 minutes. The mixture is then dried on in a tray drier at 50°C, sieved on a sieve (18 mesh) and homogenized with the indicated amount of magnesium stearate. From the homogenized mixture thus obtained tablets containing 250 mg of ciprofloxacin are pressed on a Fette EXI type single punch machine by using a concave tool having a diameter of 11 mm.

**Table VIII**

| | A | B | C | D | E |
|---|---|---|---|---|---|
| Ciprofloxacin hydrochloride monohydrate | 291.0 | 291.0 | 291.0 | 291.0 | 291.0 |
| Maltodextrin | 20.0 | 10.0 | 10.0 | 15.0 | 20.0 |
| Silicium dioxide | 20.0 | 20.0 | 14.0 | 20.0 | 14.0 |
| Sodium carboxy methyl starch | 40.0 | 50.0 | 33.0 | 45.0 | 20.0 |
| Magnesium stearate | 3.0 | 3.0 | 3.0 | 3.0 | 3.0 |
| Weight of tablet (mg) | 374.0 | 374.0 | 344.0 | 364.0 | 338.0 |
| Breaking strength (N) | 111.4 | 98.4 | 86.7 | 84.8 | 114.6 |
| Disintegration time (minutes) | 2.6 | 0.8 | 0.8 | 1.5 | 3.0 |

| Dissolution (%) | | | | | |
|---|---|---|---|---|---|
| 5 minutes | 93.5 | 90.7 | 89.8 | 82.2 | 91.3 |
| 10 minutes | 96.6 | 98.8 | 97.0 | 94.4 | 97.2 |
| 15 minutes | 97.5 | 97.2 | 97.4 | 94.9 | 97.3 |
| 30 minutes | 98.2 | 99.6 | 100.0 | 98.7 | 99.1 |

### Example 6

### Pilot plant manufacturing process

Into the 100 litres container of an Engelsmann type drum mixer 14.55 kg of ciprofloxacin hydrochloride monohydrate, 1.0 kg of maltodextrin, 2.05 kg of sodium carboxy methyl starch and 1.0 kg of colloidal silicium dioxide are weighed in and the mixture is homogenized for 15 minutes. The homogenized mixture is granulated in a Glatt WSG 15 type fluidization granulating apparatus by spraying 10.0 kg of ion-exchanged water on the mixture. The granules are dried. The dry granules are sieved on a sieve (18 mesh) and homogenized in the 100 litres container of an Engelsmann type drum mixer with 0.15 kg of magnesium stearate. One-third portions each of the homogenized mixture thus obtained are pressed on a Kilian RTS 21D type tabletting machine by using tools of appropriate size into tablets weighing 750 mg, 375 mg and 150 mg, respectively and containing 500 mg, 250 mg and 100 mg of ciprofloxacin, respectively. In a Glatt GC400 type coating equipment an aqueous suspension of 5 kg of a white Opadry Y-1-7000 type coating agent (manufacturer Colorcon Ltd.) is applied onto the tablets. Thus the tablets are coated with a layer of 16 mg, 8 mg and 4 mg, respectively. The composition of the tablets is shown in Table IX.

**Table IX**

| | 500 mg | 250 mg | 100 mg |
|---|---|---|---|
| Ciprofloxacin hydrochloride monohydrate | 582.0 | 291.0 | 116.4 |
| Sodium carboxy methyl starch | 82.0 | 41.0 | 16.4 |
| Maltodextrin | 40.0 | 20.0 | 8.0 |
| Silicium dioxide | 40.0 | 20.0 | 8.0 |
| Magnesium stearate | 6.0 | 3.0 | 1.2 |
| Weight of tablets (mg) | 750.0 | 375.0 | 350.0 |
| Opady Y-1-7000 | 16.0 | 8.0 | 4.0 |
| Weight of coated tablets (mg) | 766.0 | 383.0 | 354.0 |
| Breaking strength (N) | 123.0 | 127.0 | 124.0 |
| Disintegration time (minutes) | 4.0 | 4.0 | 10.0 |

| Dissolution (%) | | | |
|---|---|---|---|
| 5 minutes | 94.3 | 86.8 | 87.7 |
| 15 minutes | 95.4 | 92.4 | 93.1 |
| 30 minutes | 98.5 | 99.3 | 99.8 |

## Claims

1. Immediate release pharmaceutical composition containing ciprofloxacin, a binder, disintegrating agent and optionally further pharmaceutical auxiliary agents which comprises - related to the total weight of the composition -60-80 % by weight of ciprofloxacin in the form of ciprofloxacin hydrochloride monohydrate, 2-10 % by weight of maltodextrin, 5-15 % by weight of a disintegrating agent of carboxy methyl starch type, 3-6 % by weight of silicium dioxide, 1-3 % by weight of a lubricant and - in case of film-coated tablets - 2-6 % of a film coating layer.

2. Pharmaceutical composition according to Claim 1 comprising sodium carboxy methyl starch as disintegrating agent of carboxy methyl starch type.

3. Pharmaceutical composition according to Claim 1 or 2 comprising magnesium stearate as lubricant.

4. Film-coated tablet according to any of Claims 1-3 comprising on the surface of the tablets 2-6 % by weight of a water soluble film-coating layer based on hydroxy propyl methyl cellulose.

5. Process for the preparation of an immediate release pharmaceutical composition according to Claim 1 which comprises admixing - related to the total weight of the composition - 60-80 % by weight of ciprofloxacin in the form of ciprofloxacin hydrochloride monohydrate, 2-10 % by weight of maltodextrin, 5-15 % by weight of a disintegrating agent of the carboxy methyl starch type and 3-6 % by weight of silicium dioxide, granulating the mixture under the addition of water, drying, homogenizing with 1-3 % by weight of a lubricant, and pressing the mixture into tablets and, if desired, applying onto the surface of the tablets a film-coating layer in an amount of 2-6 % by weight.

6. Process according to Claim 5 which comprises using sodium carboxy methyl starch as disintegrating agent of carboxy methyl starch type.

7. Process according to Claim 5 or 6 which comprise s using magnesium stearate as lubricant.

8. Process according to any of Claims 5-7 which comprises applying on the surface of the tablets 2-6 % by weight of a water soluble film-coating layer based on hydroxy propyl methyl cellulose.

## Patentansprüche

1. Pharmazeutische Zusammensetzung zur sofortigen Freisetzung, die Ciprofloxacin, ein Bindemittel, ein Zerfallsmittel und gegebenenfalls weitere pharmazeutische Hilfsmittel enthält, die umfasst - bezogen auf das Gesamtgewicht der Zusammensetzung - 60-80 Gew.% Ciprofloxacin in der Form des Ciprofloxacinhydrochloridmonohydrats, 2-10 Gew.% Maltodextrin, 5-15 Gew.% eines Zerfallsmittels vom Carboxymethylstärke-Typ, 3-6 Gew.% Siliziumdioxid, 1-3 Gew.% eines Schmiermittels und - im Fall filmbeschichteter Tabletten - 2-6 % einer Filmbeschichtungsschicht.

2. Pharmazeutische Zusammensetzung nach Anspruch 1, die Natriumcarboxymethylstärke als Zerfallsmittel vom Carboxymethylstärke-Typ umfasst.

3. Pharmazeutische Zusammensetzung nach Anspruch 1 oder 2, die Magnesiumstearat als Schmiermittel umfasst.

4. Filmbeschichtete Tablette nach einem der Ansprüche 1-3, die auf der Oberfläche der Tabletten 2-6 Gew.% einer wasserlöslichen Filmbeschichtungsschicht auf Basis von Hydroxypropylmethylzellulose umfasst.

5. Verfahren zur Herstellung einer pharmazeutischen Zusammensetzung zur sofortigen Freisetzung nach Anspruch 1, das umfasst das Mischen - bezogen auf das Gesamtgewicht der Zusammensetzung - von 60-80 Gew.% Ciprofloxacin in Form von Ciprofloxacinhydrochloridmonohydrat, 2-10 Gew. % Maltodextrin, 5-15 Gew.% eines Zerfallsmittels vom Carboxymethylstärke-Typ und 3-6 Gew.% Siliziumdioxid, Granulieren der Mischung unter Zugabe von Wasser, Trocknen, Homogenisieren mit 1-3 Gew.% eines Schmiermittels und Pressen der Mischung in Tabletten und, falls gewünscht, Aufbringen einer Filmbeschichtungsschicht in einer Menge von 2-6 Gew.% auf die Oberfläche der Tabletten.

6. Verfahren nach Anspruch 5, das das Verwenden von Natriumcarboxymethylstärke als Zerfallsmittel vom Carboxymethylstärke-Typ umfasst.

7. Verfahren nach Anspruch 5 oder 6, das das Verwenden von Magnesiumstearat als Schmiermittel umfasst.

8. Verfahren nach einem der Ansprüche 5-7, das das Aufbringen einer wasserlöslichen Filmbeschichtungsschicht auf Basis von Hydroxypropylmethylzellulose in einer Menge von 2-6 Gew. % auf die Oberfläche der Tabletten umfasst.

## Revendications

1. Une composition pharmaceutique à libération immédiate contenant de la ciprofloxacine, un liant, un agent de désintégration et le cas échéant, d'autres agents auxiliaires pharmaceutiques qui comprend, exprimé par rapport au poids total de la composition, 60 à 80 % en poids de ciprofloxacine sous la forme de chlorhydrate de ciprofloxacine monohydraté, de 2 à 10 % en poids de maltodextrine, 5 à 15 % en poids d'un agent désintégrant de type carboxy méthyl amidon, 3 à 6 % en poids de dioxyde de silicium, 1 à 3 % en poids d'un lubrifiant, et, dans le cas de comprimés enrobés d'un film, 2 à 6 % d'une couche d'enrobage pelliculaire.

2. Composition pharmaceutique selon la revendication 1, comprenant du carboxy méthyl amidon sodique en tant qu'agent de désintégration de type carboxy méthyl amidon.

3. Composition pharmaceutique selon la revendication 1 ou 2, comprenant du stéarate de magnésium en tant que lubrifiant.

4. Comprimé enrobé de film selon l'une quelconque des revendications 1 à 3, comprenant à la surface des comprimés de 2 à 6 % en poids d'une couche d'enrobage pelliculaire hydrosoluble à base d'hydroxy propyl méthyl cellulose.

5. Procédé de préparation d'une composition pharmaceutique à libération immédiate selon la revendication 1, qui comprend le mélange, rapporté au poids total de la composition, de 60 à 80 % en poids de ciprofloxacine sous forme de chlorhydrate de ciprofloxacine monohydraté, de 2 à 10 % en poids de maltodextrine, 5 à 15 % en poids d'un agent de désintégration de type carboxy méthyl amidon, et 3 à 6 % en poids de dioxyde de silicium, la granulation du mélange sous addition d'eau, le séchage, l'homogénéisation avec 1 à 3 % en poids d'un lubrifiant et la compression du mélange en comprimés et, le cas échéant, application sur la surface des comprimés d'une couche d'enrobage pelliculaire à raison de 2 à 6 % en poids.

6. Le procédé selon la revendication 5, qui comprend l'utilisation du carboxy méthyl amidon sodique en tant qu'agent de désintégration de type carboxy méthyl amidon.

7. Le procédé selon la revendication 5 ou 6 qui comprend l'utilisation de stéarate de magnésium en tant que lubrifiant.

8. Le procédé selon l'une quelconque des revendications 5 à 7 qui comprend l'application à la surface des comprimés de 2 à 6 % en poids d'une couche d'enrobage pelliculaire hydrosoluble à base d'hydroxy propylméthyl cellulose.
